Europäisches Patentamt

European Patent Office

Office européen des brevets

(11) Veröffentlichungsnummer: **0 244 391**
**A2**

(12) **EUROPÄISCHE PATENTANMELDUNG**

(21) Anmeldenummer: **87890053.9**

(22) Anmeldetag: **18.03.87**

(51) Int. Cl.⁴: **C 05 F 9/02**
**A 61 L 9/00, A 61 L 11/00**

(30) Priorität: **30.04.86 AT 1176/86**

(43) Veröffentlichungstag der Anmeldung:
**04.11.87 Patentblatt 87/45**

(84) Benannte Vertragsstaaten:
**BE CH DE ES FR GB GR IT LI LU NL SE**

(71) Anmelder: **VOEST-ALPINE Aktiengesellschaft**
**Friedrichstrasse 4**
**A-1011 Wien (AT)**

(72) Erfinder: **Klinar, Gottfried**
**Alpenstrasse 33**
**A-8707 Leoben (AT)**

(74) Vertreter: Haffner, Thomas M., Dr. et al
**Patentanwaltskanzlei Dipl.-Ing. Adolf Kretschmer Dr.**
**Thomas M. Haffner Schottengasse 3a**
**A-1014 Wien (AT)**

(54) Verfahren zum Belüften einer Rotte sowie Vorrichtung zur Durchführung dieses Verfahrens.

(57) Um den kontinuierlichen Betrieb eines biologischen Filters unter konstanten Bedingungen sicherzustellen, wird die zum Belüften einer Rotte (1) durch die Rotte (1) hindurchgesaugte Luft einem Wasch- und/oder Kühlaggregat (5) zugeführt, in welchem die Temperatur herabgesenkt wird und die Feuchtigkeit des Gases auf die jeweils gewünschten Betriebsbedingungen im Kompostfilter (9) eingestellt wird. Das Waschen und/oder Kühlen des Gases erfolgt nach einem Gebläse (4), so daß auch der Temperaturerhöhung durch das Gebläse (4) Rechnung getragen wird und eine Absenkung der Temperatur des Gases auf die für das Kompostfilter (9) gewünschten Werte möglich wird.

EP 0 244 391 A2

**Beschreibung**

Verfahren zum Belüften einer Rotte sowie Vorrichtung zur Durchführung dieses Verfahrens

Die Erfindung bezieht sich auf ein Verfahren zum Belüften einer Rotte, bei welchem Luft durch die Rotte gesaugt und vor dem Ausstoß in die Atmosphäre über ein biologisches Filter, beispielsweise ein Kompostfilter, geleitet wird, sowie auf eine Vorrichtung zur Durchführung dieses Verfahrens.

Die zum Belüften einer Rotte durch einzelne Rottezellen hindurchgesaugte Luft ist prozeßbedingt stets wasserdampfgesättigt und weist 100 % relative Luftfeuchtigkeit bei einer Temperatur auf, welche üblicherweise zwischen 60 und 70°C beträgt. Mit der durch die Rotte hindurchgesaugten Luft werden eine Reihe organischer Substanzen mittransportiert, welche in der Folge zum Teil als übelriechende Stoffe besonders störend wirken. Es ist bekannt, die durch die Rotte hindurchgesaugte Luft vor dem Ausstoßen in die Atmosphäre durch ein Filter zu schicken und es sind zu diesem Zweck Erdfilter und Sandfilter vorgeschlagen worden. Es ist weiters bereits vorgeschlagen worden, den organischen Anteil, welcher für die Geruchsbelästigung verantwortlich ist, biologisch abzubauen und zu diesem Zweck wurde bereits vorgeschlagen, die Luft durch ein Kompostfilter zu schicken, welcher mit bereits fertigem Kompost beschickt ist. Derartige Kompostfilter sollen es ermöglichen, den mit der Luft mitgerissenen organischen Anteil mikrobiell abzubauen, so daß eine von organischen Bestandteilen gereinigte und im wesentlichen geruchsfreie Luft das Filter verlassen kann. In der Praxis hat sich nun gezeigt, daß derartige Filter nicht immer eine ausreichende Betriebssicherheit aufweisen und nur relativ geringe Standzeiten haben und ihre Aufgabe nach kürzerer Zeit und vor allen Dingen nach Betriebsunterbrechungen keineswegs mehr erfüllen können.

Die Erfindung zielt nun darauf ab, ein Verfahren der eingangs genannten Art zu schaffen, welches es ermöglicht, Betriebssicherheit und Standzeit eines biologischen Filters wesentlich zu erhöhen und mit ein und dergleichen Filterschüttung große Mengen an durch das Rottegut durchgesaugter Luft zuverlässig zu reinigen und geruchsfrei zu machen. Zur Lösung dieser Aufgabe besteht das erfindungsgemäße Verfahren im wesentlichen darin, daß die durch die Rotte gesaugte Luft vor dem Einleiten in das biologische Filter gewaschen und/oder gekühlt wird. Es hat sich gezeigt, daß biologische Filter, insbesondere mit Kompost beschickte Filter dann optimal arbeiten, wenn die Feuchte innerhalb des Filters relativ konstant gehalten werden kann und die Temperatur des Filters zwischen 30 und 40° gehalten werden kann. Da die durch die Rotte hindurchgesaugte Luft üblicherweise Temperaturen von 60 bis 70° aufweist, bewirkt die Kühlung eine Absenkung der Temperatur und damit eine Verbesserung der Fähigkeit der Mikroben im Kompostfilter, die organischen Bestandteile umzusetzen. Durch die Kühlung und/oder Waschung des Gases läßt sich aber auch der Feuchtigkeitsgehalt auf die gewünschten niedrigeren Werte bringen und es kann auf diese Weise verhindert werden, daß das nachgeschaltete Kompostfilter überfeuchtet wird. Während im Rottebetrieb Schwankungen der Temperatur prozeßbedingt auftreten müssen, läßt sich durch ein Waschen und/oder Kühlen vor dem Einleiten des abgesaugten Gases in das Kompostfilter ein Ausgleich derartiger Temperaturschwankungen herbeiführen, wodurch annähernd Temperaturkonstanz innerhalb des Filters erzielt werden kann. Das Waschen und Kühlen ermöglicht in einfacher Weise auch das Abscheiden von Nebel aus dem durchgesaugten Gas, so daß der Feuchtigkeitsgehalt jeweils auf die gewünschten Werte eingestellt werden kann. In besonders einfacher Weise wird das erfindungsgemäße Verfahren so durchgeführt, daß die durch die Rotte gesaugte Luft nach dem Sauggebläse auf 30 bis 45°C gekühlt wird.

Zur Verminderung der Restfeuchte wird vorzugsweise vor dem Sauggebläse und gegebenenfalls nach dem Sauggebläse sich bildender Nebel abgeschieden. Das Abscheiden von sich bildendem Nebel vor dem Sauggebläse dient hiebei der Schonung des Sauggebläses und die Kühlung und/oder Waschung nach dem Sauggebläse trägt dem Umstand Rechnung, daß durch das Sauggebläse selbst noch eine weitere Temperaturerhöhung des durch die Rotte hindurchgesaugten Gases eintritt.

Das erfindungsgemäße Verfahren läßt sich in besonders einfacher Weise regeln und es wird zu diesem Zweck mit Vorteil so vorgegangen, daß die Temperatur und/oder der Wassergehalt des biologischen Filters gemessen wird und in Abhängigkeit von diesen Meßwerten die Kühlung und/oder Entfeuchtung des Luftstromes nach dem Sauggebläse geregelt wird.

Eine besonders einfache Vorrichtung zur Durchführung des erfindungsgemäßen Verfahrens ist im wesentlichen dadurch gekennzeichnet, daß in die Luftleitung zwischen Rotte und Filter in Strömungsrichtung nach der Pumpe bzw. dem Ventilator ein Wasch- und/oder Kühlaggregat eingeschaltet ist, welches zusätzlich zu den Anschlüssen für die Luftleitung einen Anschluß für das Ableiten von kondensierendem und/oder zusätzlich eingeleitetem Wasser aufweist. Ein derartiges Wasch- und/oder Kühlaggregat kann in einfacher Weise als Turm ausgebildet sein und zum Zwecke des Waschens des Gases eine Einrichtung zum Überbrausen von im Gegenstrom geführter Luft aufweisen. Das Überbrausen mit Wasser kann hiebei einesteils zur Einstellung einer konstanten Feuchtigkeit und andernteils zur Kühlung des Gasstromes herangezogen werden, wobei zum Zwecke einer intensiven Kühlung jeweils Frischwasser zugeführt werden kann. Um eine Temperaturregelung zu erzielen, kann nun mit Vorteil das Wasch- bzw. Kühlwasser des Turmes zumindest teilweise über eine Pumpe im Kreislauf geführt sein, wobei der im Kreislauf geführte Anteil des Wasch- bzw. Kühlwassers naturgemäß eine höhere Temperatur aufweist als das Frischwasser. Durch Zudosieren von Frischwasser oder durch Erhöhen des Anteiles der im Kreislauf geführten Wasch- bzw. Kühlflüssigkeit läßt sich das gewünschte Temperaturniveau für das nachfolgende biologische Filter einstellen.

Zum Zwecke des Einregelns bestimmter Temperatur- bzw. bestimmter Feuchtigkeitsgehalte der dem biologischen Filter zuzuführenden Luft und insbesondere zur Erzielung konstanter Verhältnisse im biologischen Filter, welche es ermöglichen die Filter stationär zu betreiben, sind mit Vorteil im biologischen Filter Sensoren zum Erfassen der Temperatur und/oder der Feuchtigkeit angeordnet, deren Meßwerte über ein Schaltwerk den steuerbaren Ventilen für die Frischwasserzufuhr und/oder die Kreislaufpumpe des Kühl- und/oder Waschturmes zugeführt sind. Im Sumpf des Kühl- und/oder Waschturmes sammelt sich das Kühl- und/oder Waschwasser sowie das aufgrund der Temperaturerniedrigung kondensierende Wasser aus dem feuchten Gas und es kann mit Vorteil mit dem Sumpf des Wasch- und/oder Kühlaggregates ein Mehrwegeventil verbunden sein. Ein derartiges Mehrwegeventil erlaubt es, in einer Stellung die Flüssigkeit des Sumpfes der Kreislaufpumpe zuzuführen und auf diese Weise temperiertes Wasser zur Regelung rückzuführen. Andererseits erlaubt es das Mehrwegeventil, das im Sumpf enthaltene Wasser auszubringen, sobald der Anteil an im Wasser gelösten organischen Substanzen kritische Grenzwerte erreicht. Zu diesem Zweck sind mit Vorteil in die Kreislaufleitung für das Wasch- und/oder Kühlwasser Sensoren für die Erfassung des pH-Wertes und/oder des Anteiles organischer Säuren eingeschaltet, deren Meßwerte über ein Schaltwerk dem steuerbaren Mehrwegeventil in der Kreislaufleitung des Wasch- und/oder Kühlaggregates zugeführt sind. Eine besonders einfache Ausbildung derartiger Sensoren stellen Leitfähigkeitsmeßgeräte dar.

Zur Verbesserung der Temperaturregelung ist mit Vorteil die Ausbildung so getroffen, daß das Wasch- und/oder Kühlaggregat Füllkörper, insbesondere keramische Hohlkörper, enthält. Derartige Füllkörper befinden sich nach kurzzeitigem Betrieb der Vorrichtung auf einem bestimmten Temperaturniveau, welches selbst wiederum eine Speicherwirkung ausübt und auf diese Weise zur Konstanz der Temperatur des das Wasch- und/oder Kühlaggregat verlassenden Gases beiträgt.

Mit dem Waschwasser werden in der Regel die meisten geruchsintensiven organischen Substanzen bereits eliminiert, da diese geruchsintensiven Stoffe zumeist sehr gut wasserlöslich sind bzw. ähnliche Taupunkte wie das Wasser aufweisen. Die Temperaturabsenkung und die Einstellung des Feuchtigkeitsgehaltes erlaubt es, einen stationären Betrieb des biologischen Filters sicherzustellen, wobei gleichzeitig ein Teil der organischen Substanz, insbesondere organische Säuren, bereits vor dem Eintreten in das biologische Filter eliminiert werden, wodurch Standzeit und Betriebssicherheit des Filters weiter erhöht werden können.

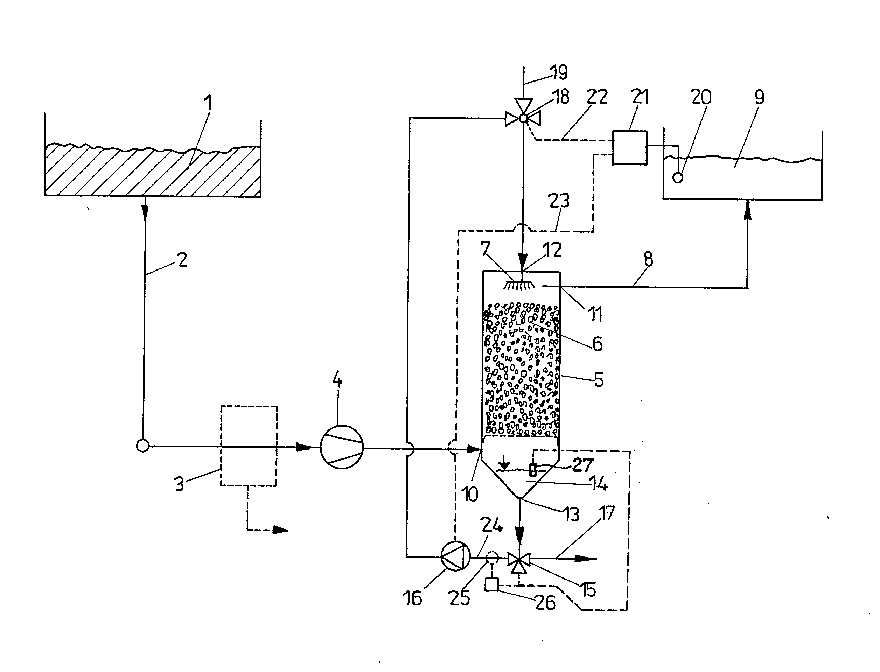

Die Erfindung wird nachfolgend anhand eines in der Zeichnung schematisch dargestellten Ausführungsbeispieles einer für die Durchführung des erfindungsgemäßen Verfahrens geeigneten Vorrichtung näher erläutert.

In der Zeichnung ist mit 1 die Rotte bezeichnet. Über eine Leitung 2 wird durch die Rotte Luft hindurchgesaugt, um die Rotte zu belüften. Die Luft verläßt die Rotte 1 mit Temperaturen, vorzugsweise zwischen 60 und 70°C, wobei Sättigungsbedingungen bezüglich des mit der Luft mittransportierten Wassers vorherrschen. Mit 3 ist ein fakultativ vorgesehener Nebelabscheider angedeutet, durch welchen überschüssige Feuchtigkeit vor dem Eintritt in ein Gebläse 4 eliminiert werden kann. Durch das Gebläse 4 wird der Saugdruck eingestellt und das komprimierte und wiederum geringfügig erwärmte Gas gelangt in einen Wasch- und/oder Kühlturm 5, welcher mit Füllkörpern 6 gefüllt ist. Die Gasführung erfolgt im Gegenstrom zu einer Waschflüssigkeit, welche über eine Brause 7 am Kopfende des Turmes zugeführt werden kann. Das gereinigte und entsprechend abgekühlte Gas tritt über die Leitung 8 aus und gelangt in ein mit Kompost gefülltes biologisches Filter 9. Nach dem Durchtritt durch das biologische Filter 9 tritt gereinigte und im wesentlichen desodorierte Luft in die Atmosphäre aus.

Das Wasch- und/oder Kühlaggregat 5 weist neben den Anschlüssen 10 und 11 für das Gas einen Anschluß 12 für die Zufuhr von Waschflüssigkeit und einen Anschluß 13 für das Ableiten von kondensierendem Wasser bzw. von Waschwasser aus dem Sumpf 14 auf. In die Abwasserleitung ist ein Dreiwegeventil 15 eingeschaltet, über welches Waschflüssigkeit bzw. kondensierendes Wasser einer Kreislaufpumpe 16 zugeführt werden kann oder aber über die Leitung 17 abgezogen werden kann. Die Frischwasserzufuhr erfolgt gleichfalls über ein sperrbares Ventil 18, wobei die Frischwasserleitung mit 19 bezeichnet ist.

Im Kompostfilter 9 sind Sensoren 20 angeordnet, deren Meßwerte einem, beispielsweise einen Mikroprozessor enthaltenden, elektronischen Schaltwerk 21 zugeführt werden. Über Steuerleitungen 22 und 23 kann von diesem Schaltwerk 21 der Anteil des Frischwassers sowie der Anteil der im Kreislauf geführten Flüssigkeitsmenge durch Regelung der Pumpe 16 verändert werden, wodurch die Kühlung beeinflußt werden kann. In die Abwasserleitung 24 ist ein Sensor 25 eingeschaltet, welcher den Grad der Verschmutzung des im Kreislauf geführten Wassers, beispielsweise durch Messung des pH-Wertes, ermittelt. Die Meßwerte dieses Sensors 25 werden über ein Schaltwerk 26 dem steuerbaren Dreiwegeventil 15 als Steuerbefehle zur Verfügung gestellt, wodurch bei zu großer Verschmutzung die Ableitung des Wassers über die Leitung 17 vorgenommen wird, worauf neues Frischwasser im Kreislauf geführt wird bis die Verschmutzung kritische Grenzwerte erreicht. Weiters kann mittels eines Sensors 27 Niveaumessung im Sumpf 14 vorgesehen sein, wobei bei Erreichen eines maximalen Niveaus ein Ablassen des Kreislaufwassers über das Dreiwegeventil 15 und die Leitung 17 vorgenommen werden kann.

Mit dem Gebläse 4 werden Saugdrücke von 1 bis 3 m Wassersäule erreicht, wodurch sich eine Steigerung der Temperatur des Gases und eine Absenkung der relativen Luftfeuchtigkeit ergibt. Das dem Kompostfilter 9 zuzuführende Gas soll den stationären Betrieb dieses Filters bei Temperaturen, vorzugsweise zwischen 30 und 40° und einer relativ hohen Luftfeuchtigkeit sicherstellen, welche mit Vorteil zwischen 96 und 99 % relativ eingestellt werden soll. Unter 96 % relativer Luftfeuchtigkeit hat es sich gezeigt, daß die im Kompostfilter enthaltenen Mikroben ihre Tätigkeit und damit die Umsetzung organischer Substanzen einstellen. Gleiches gilt für zu große Temperaturschwankungen, welche zu einer Inaktivierung des Kompostfilters führen. Zu hohe Luftfeuchtigkeit im Kompostfilter führt dazu, daß Wasser im Filter verbleibt, wodurch gleichfalls die Funktionsfähigkeit des Filters beeinträchtigt wird und nach kurzer Zeit ein Durchbrechen des Filters beobachtet wird.

**Patentansprüche**

1. Verfahren zum Belüften einer Rotte (1), bei welchem Luft durch die Rotte gesaugt und vor dem Ausstoß in die Atmosphäre über ein biologisches Filter (9), z.B. ein Kompostfilter, geleitet wird, dadurch gekennzeichnet, daß die durch die Rotte (1) gesaugte Luft vor dem Einleiten in das biologische Filter (9) gewaschen und/oder gekühlt wird.

2. Verfahren nach Anspruch 1, dadurch gekennzeichnet, daß die durch die Rotte (1) gesaugte Luft nach dem Sauggebläse (4) auf 30-45°C gekühlt wird.

3. Verfahren nach Anspruch 1 oder 2, dadurch gekennzeichnet, daß vor dem Sauggebläse (4) und gegebenenfalls nach dem Sauggebläse (4) und/oder nach der Kühl- bzw. Waschstrecke (5) sich bildender Nebel abgeschieden wird.

4. Verfahren nach Anspruch 1, 2 oder 3, dadurch gekennzeichnet, daß die Temperatur und/oder der Wassergehalt des biologischen Filters (9) gemessen wird und in Abhängigkeit von diesen Meßwerten die Kühlung und/oder Entfeuchtung des Luftstromes nach dem Sauggebläse (4) geregelt wird.

5. Vorrichtung zum Durchführen des Verfahrens nach einem der Ansprüche 1 bis 4, dadurch gekennzeichnet, daß in die Luftleitung zwischen Rotte (1) und Filter (9) in Strömungsrichtung nach der Pumpe bzw. dem Ventilator (4) ein Wasch- und/oder Kühlaggregat (5) eingeschaltet ist, welches zusätzlich zu den Anschlüssen für die Luftleitung (10, 11) einen Anschluß für das Ableiten von kondensierendem und/oder zusätzlich eingeleitetem Wasser (12, 13) aufweist.

6. Vorrichtung nach Anspruch 5, dadurch gekennzeichnet, daß das Wasch- und/oder Kühlaggregat (5) als Turm ausgebildet ist und eine Einrichtung zum Überbrausen (7) von im Gegenstrom geführter Luft aufweist.

7. Vorrichtung nach Anspruch 5 oder 6, dadurch gekennzeichnet, daß das Wasch- bzw. Kühlwasser des Turmes (5) zumindest teilweise über eine Pumpe (16) im Kreislauf geführt ist.

8. Vorrichtung nach einem der Ansprüche 5, 6 oder 7, dadurch gekennzeichnet, daß im biologischen Filter (9) Sensoren (20) zum Erfassen der Temperatur und/oder der Feuchtigkeit angeordnet sind, deren Meßwerte über ein Schaltwerk (21) den steuerbaren Ventilen (18) für die Frischwasserzufuhr (19) und/oder der Kreislaufpumpe (16) des Kühl- und/oder Waschturmes (5) zugeführt sind.

9. Vorrichtung nach einem der Ansprüche 5 bis 8, dadurch gekennzeichnet, daß mit dem Sumpf (14) des Wasch- und/oder Kühlaggregates (5) ein Mehrwegeventil (15) verbunden ist.

10. Vorrichtung nach einem der Ansprüche 5 bis 9, dadurch gekennzeichnet, daß in die Kreislaufleitung für das Waschund/oder Kühlwasser Sensoren (25) für die Erfassung des pH-Wertes und/oder des Anteiles organischer Säuren eingeschaltet sind, deren Meßwerte über ein Schaltwerk (26) dem steuerbaren Mehrwegeventil (15) in der Kreislaufleitung des Wasch- und/oder Kühlaggregates (5) zugeführt sind.

11. Vorrichtung nach einem der Ansprüche 5 bis 10, dadurch gekennzeichnet, daß das Wasch- und/oder Kühlaggregat (5) Füllkörper (6), insbesondere keramische Hohlkörper, enthält.

0244391